## Europäisches Patentamt
## European Patent Office
## Office européen des brevets

(11) Veröffentlichungsnummer: **0 324 348**
**A2**

(12) **EUROPÄISCHE PATENTANMELDUNG**

(21) Anmeldenummer: 89100033.3

(22) Anmeldetag: 03.01.89

(51) Int. Cl.⁴ **A61K 31/445**

Die Bezeichnung der Erfindung wurde geändert (Richtlinien für die Prüfung im EPA, A-III, 7.3).

(30) Priorität: 14.01.88 DE 3800868

(43) Veröffentlichungstag der Anmeldung:
19.07.89 Patentblatt 89/29

(84) Benannte Vertragsstaaten:
AT BE CH DE FR GB IT LI LU NL SE

(71) Anmelder: **Dr. Karl Thomae GmbH**
**Postfach 1755**
**D-7950 Biberach 1(DE)**

(72) Erfinder: **Eberlein, Wolfgang, Dr. Dipl.-Chem.**
**Obere Au 6**
**D-7950 Biberach 1(DE)**
Erfinder: **Engel, Wolfhard, Dr. Dipl.-Chem.**
**Mozartstrasse 13**
**D-7950 Biberach 1(DE)**
Erfinder: **Mihm, Gerhard, Dr. Dipl.-Chem.**
**Nickeleshalde 5/1**
**D-7950 Biberach 1(DE)**
Erfinder: **Mayer, Norbert, Dr.**
**Friedrich-Ebert-Strasse 66**
**D-7950 Biberach 1(DE)**
Erfinder: **de Jonge, Adriaan, Dr.**
**De Boomgaard 19**
**NL-3971 LD Driebergen(NL)**

(54) Piperidincarboxamid-Derivate zur Behandlung von Bradycardien und Bradyarrhythmien.

(57) (±)-1-[4-[Ethyl[2-(4-methoxyphenyl)-1-methylethyl]amino]-1-oxobutyl]-N,N-dimethyl-4-piperidincarboxamid und ( + )-1-[4-[Ethyl[2-(4-methoxyphenyl)-1-methylethyl]amino]-1-oxobutyl]-N,N-dimethyl-4-piperidincarboxamid, des weiteren die physiologisch verträglichen Säureadditionssalze dieser Verbindungen eignen sich zur Behandlung von Bradycardien und Bradyarrhythmien.

EP 0 324 348 A2

## Mittel zur Behandlung von Bradycardien und Bradyarrhythmien

Die Erfindung betrifft ein Mittel zur Behandlung von Bradycardien und Bradyarrhythmien, welches die Verbindungen (±)-1-[4-[Ethyl[2-(4-methoxyphenyl)-1-methylethyl]amino]-1-oxobutyl]-N,N-dimethyl-4-piperidincarboxamid (I) und (+)-1-[4-[Ethyl[2-(4-methoxyphenyl)-1-methylethyl]amino]-1-oxobutyl]-N,N-dimethyl-4-piperidincarboxamid (II) sowie deren physiologisch verträgliche Salze mit anorganischen oder organischen Säuren enthält.

In der europäischen Patentanmeldung Nr. 85 201 394.5 (Veröffentlichungs-Nr. 0 177 078) werden Verbindungen beschrieben, die als muscarinische Rezeptorantagonisten spezifische spasmolytische Eigenschaften besitzen und damit vorteilhaft zur Behandlung von Spasmen im Gastrointestinaltrakt eingesetzt werden können.

Es wurde überraschend gefunden, daß die in dem oben angeführten Patent enthaltenen Verbindungen

    I.    (±)-1-[4-[Ethyl[2-(4-methoxyphenyl)-1-methylethyl]amino]-1-oxobutyl]-N,N-dimethyl-4-piperidincarboxamid und

    II.    (+)-1-[4-[Ethyl[2-(4-methoxyphenyl)-1-methylethyl]amino]-1-oxobutyl]-N,N-dimethyl-4-piperidincarboxamid

und ihre physiologisch verträglichen Salze mit anorganischen oder organischen Säuren noch gänzlich andersgeartete pharmakologische Eigenschaften besitzen, die ihre Anwendung als vagale Schrittmacher zur Behandlung von Bradycardien und Bradyarrhythmien ermöglichen.

Für die obengenannten Verbindungen I und II wurde überraschenderweise eine ausgeprägte Selektivität für die Muskarinrezeptoren am Herzen gefunden. Der große Abstand der erwünschten Effekte auf die Herzfrequenz zu den unerwünschten anticholinergen Wirkungen ermöglicht den Einsatz der Verbindungen I und II als vagale Schrittmacher zur Behandlung verschiedener Formen von Bradycardien und Bradyarrhythmien, ohne daß mit intolerablen Nebenwirkungen zu rechnen ist.

Eine günstige Relation zwischen tachykarden Wirkungen einerseits und den bei Therapeutika mit anticholinerger Wirkkomponente auftretenden unerwünschten Wirkungen auf die Pupillenweite und Tränen-, Speichel- und Magensäuresekretion andererseits ist für die therapeutische Verwendung der Substanzen von besonderer Wichtigkeit.

Die folgenden Versuche zeigen, daß die erfindungsgemäßen Verbindungen diesbezüglich überraschend günstige Relationen aufweisen.

A Bindungsstudien an muscarinischen Rezeptoren:

### Bestimmung des $IC_{50}$-Wertes in vitro

Als Organspender dienten männliche Sprague-Dawley-Ratten mit 180-220 g Körpergewicht. Nach Entnahme von Herz, Submandibularis und Großhirnrinde wurden alle weiteren Schritte in eiskaltem Hepes-HCl-Puffer (pH 7,4; 100 mmolar NaCl, 10 mmolar $MgCl_2$) vorgenommen. Das Gesamtherz wurde mit einer Schere zerkleinert. Alle Organe wurden abschließend in einem Potter homogenisiert.

Für den Bindungstest wurden die Organhomogenate in folgender Weise verdünnt:

| | |
|---|---|
| Gesamtherz | 1 : 400 |
| Großhirnrinde | 1:3000 |
| Submandibularis | 1 : 400 |

Die Inkubation der Organhomogenate erfolgte bei einer bestimmten Konzentration des Radioliganden und einer Konzentrationsreihe der nichtradioaktiven Testsubstanzen im Eppendorf-Zentrifugenröhrchen bei 30° C. Die Inkubationsdauer betrug 45 Minuten. Als Radioligand wurde 0,3 n molar $^3$H-N-Methylscopolamin ($^3$H-NMS) verwendet. Die Inkubation wurde durch Zugabe von eiskaltem Puffer mit nachfolgender Vakuumfiltration beendet. Die Filter wurden mit kaltem Puffer gespült und ihre Radioaktivität bestimmt. Sie repräsentiert die Summe von spezifischer und unspezifischer Bindung von $^3$H-NMS. Der Anteil der unspezifischen Bindung wurde definiert als jene Radioaktivität, die in Anwesenheit von 1 µmolar Quinuclidinylbenzilat gebunden wurde. Es wurden immer Vierfachbestimmungen vorgenommen. Die $IC_{50}$-Werte der

nichtmarkierten Testsubstanzen wurden graphisch bestimmt. Sie repräsentieren jene Konzentration der Testsubstanz, bei welcher die spezifische Bindung von ³H-NMS an die muscarinischen Rezeptoren in den verschiedenen Organen um 50 % gehemmt wurde. Die Ergebnisse sind aus der Tabelle 1 zu ersehen.

B. Untersuchung auf funktionelle Selektivität der antimuscarinischen Wirkung

Substanzen mit antimuscarinischen Eigenschaften inhibieren die Wirkungen von exogen zugeführten Agonisten oder von Acetylcholin, das aus cholinergen Nervenendigungen freigesetzt wird. Im folgenden wird die Beschreibung einer "in vivo"-Methode wiedergegeben, die zur Erfassung von cardioselektiven Antimuscarinica geeignet ist. Die angewandte Methode hatte zum Ziel, die Selektivität der antimuscarinischen Wirkung zu bestätigen.

Hemmung der Acetylcholinwirkung auf Blase, Bronchien und Herzfrequenz des Meerschweinchens

Am narkotisierten Meerschweinchen wurden 5 Minuten nach Gabe der Prüfsubstanz 10 μg/kg Acetylcholin intravenös und gleichzeitig intraarteriell injiziert. Dabei wurde die Herzfrequenz durch extrakorporale Ableitung des EKG, der Ausatemwiderstand nach Konzett-Rößler und die Kontraktion der freigelegten Harnblase direkt registriert. Für die Hemmung der Acetylcholinwirkung an den untersuchten Organen wurden Dosis-Wirkungskurven aufgenommen und daraus $-\log\text{-}ED_{50}$-Werte bestimmt. Ergebnisse siehe Tabelle II.

Nach den vorstehenden Angaben wurden beispielsweise die folgenden Verbindungen untersucht:

A = (±)-1-[4-[Ethyl[2-(4-methoxyphenyl)-1-methylethyl]amino]-1-oxobutyl]-N,N-dimethyl-4-piperidin-carboxamid und

B = Atropin.

Tabelle I:

| Rezeptor-Bindungs-Tests, in vitro: | | | |
|---|---|---|---|
| Ergebnisse: | | | |
| Substanz | Rezeptor-Bindungs-Tests $IC_{50}[nMl^{-1}]$ | | |
| | Cortex | Herz | Submandibularis |
| A | 50 | 10 | 300 |
| B | 2 | 4 | 4 |

Die Angaben in der vorstehenden Tabelle I beweisen, daß die neue Verbindung zwischen muscarinischen Rezeptoren verschiedener Gewebe unterscheidet. Dies folgt aus den beträchtlich niedrigeren $IC_{50}$-Werten bei Untersuchung an Präparaten aus dem Herzen gegenüber solchen aus der Großhirnrinde. Insbesondere geht aber aus den Bindungsdaten hervor, daß die Herzfrequenz durch die genannte Verbindung bei Dosierungen gesteigert wird, bei denen noch keine Einschränkung der Speichelsekretion zu erwarten ist.

3

Tabelle II:

| Hemmung der Acetylcholinwirkung auf Blase, Bronchien und Herzfrequenz des Meerschweinchens: | | | |
|---|---|---|---|
| Ergebnisse: | | | |
| Substanz | -log $ED_{50}$ [Molkg$^{-1}$] | | |
| | Herz | Bronchien | Blase |
| A | 7.19 | 6.83 | 6.29 |

Die pharmakologischen Daten der vorstehenden Tabelle II deuten auf ein überraschend großes Unterscheidungsvermögen zwischen Herz und glatter Muskulatur der Harnblase hin.

Zur pharmazeutischen Anwendung lassen sich die Verbindungen I und II in an sich bekannter Weise in die üblichen pharmazeutischen Zubereitungsformen, z.B. in Lösungen, Suppositorien, Tabletten, Dragées, Kapseln oder Teezubereitungen einarbeiten. Die Tagesdosis liegt im allgemeinen zwischen 0,02 und 5 mg/kg, vorzugsweise 0,02 und 2,5 mg/kg, insbesondere 0,05 und 1,0 mg/kg Körpergewicht, die gegebenenfalls in Form mehrerer, vorzugsweise 1 bis 3 Einzelgaben, zur Erzielung der gewünschten Ergebnisse verabreicht wird.

Die Herstellung der Verbindungen I und II wird in der europäischen Patentanmeldung Nr. 85 201 394.5 (Veröffentlichungs-Nr. 0 177 078) beschrieben.

Die nachfolgenden Beispiele beschreiben die Herstellung einiger pharmazeutischer Zubereitungsformen:

Beispiel I

Tabletten mit 20,0 mg (±)-1-[4-[Ethyl[2-(4-methoxyphenyl)-1-methylethyl]amino]-1-oxobutyl]-N,N-dimethyl-4-piperidincarboxamid

| Zusammensetzung: | |
|---|---|
| 1 Tablette enthält: | |
| Wirkstoff | 20,0 mg |
| Milchzucker | 152,0 mg |
| Kartoffelstärke | 65,0 mg |
| Magnesiumstearat | 2,0 mg |
| | 239,0 mg |

Herstellungsverfahren:

Aus Kartoffelstärke wird durch Erwärmen ein 10%iger Schleim hergestellt. Die Wirksubstanz, Milchzucker und die restliche Kartoffelstärke werden gemischt und mit obigem Schleim durch ein Sieb der Maschenweite 1,5 mm granuliert. Das Granulat wird bei 45° C getrocknet, nochmals durch obiges Sieb gerieben, mit Magnesiumstearat vermischt und zu Tabletten verpreßt.

| Tablettengewicht: | 239 mg |
|---|---|
| Stempel: | 9 mm |

Beispiel II

Dragées mit 20,0 mg (±)-1-[4-[Ethyl[2-(4-methoxyphenyl)-1-methylethyl]amino]-1-oxobutyl]-N,N-dimethyl-4-piperidincarboxamid

Die nach Beispiel I hergestellten Tabletten werden nach bekanntem Verfahren mit einer Hülle überzogen, die im wesentlichen aus Zucker und Talkum besteht. Die fertigen Dragées werden mit Hilfe von Bienenwachs poliert.

Dragéegewicht: 300 mg

Beispiel III

Ampullen mit 4,0 mg (±)-1-[4-[Ethyl[2-(4-methoxyphenyl)-1-methylethyl]amino]-1-oxobutyl]-N,N-dimethyl-4-piperidincarboxamid

| Zusammensetzung: | |
|---|---|
| 1 Ampulle enthält: | |
| Wirkstoff | 4,0 mg |
| Natriumchlorid | 8,0 mg |
| Dest. Wasser ad | 1 ml |

Herstellungsverfahren

Die Wirksubstanz und Natriumchlorid werden in dest. Wasser gelöst und anschließend auf das gegebene Volumen aufgefüllt. Die Lösung wird sterilfiltriert und in 1ml-Ampullen abgefüllt.

Sterilisation: 20 Minuten bei 120° C.

Beispiel IV

Suppositorien mit 20,0 mg (±)-1-[4-[Ethyl[2-(4-methoxyphenyl)1-methylethyl]amino]-1-oxobutyl]-N,N-dimethyl-4-piperidin-carboxamid

| Zusammensetzung: | |
|---|---|
| 1 Zäpfchen enthält: | |
| Wirkstoff | 20,0 mg |
| Zäpfchenmasse (z.B. Witepsol W 45®) | 1690,0 mg |
| | 1710,0 mg |

Herstellungsverfahren:

Die feinpulverisierte Wirksubstanz wird in der geschmolzenen und auf 40° C abgekühlten Zäpfchenmasse suspendiert. Man gießt die Masse bei 37° C in leicht vorgekühlte Zäpfchenformen aus.

Zäpfchengewicht: 1,71 g

5

Beispiel V

Tropfen mit (±)-1-[4-[Ethyl[2-(4-methoxyphenyl)1-methylethyl]amino]-1-oxobutyl]-N,N-dimethyl-4-piperidin-carboxamid

| Zusammensetzung: | |
|---|---|
| 100 ml Tropflösung enthälten: | |
| p-Hydroxybenzoesäuremethylester | 0,035 g |
| p-Hydroxybenzoesäurepropylester | 0,015 g |
| Anisöl | 0,05 g |
| Menthol | 0,06 g |
| Ethanol rein | 10,0 g |
| Wirkstoff | 0,8 g |
| Natriumcyclamat | 1,0 g |
| Glycerin | 15,0 g |
| Dest. Wasser ad | 100,0 ml |

Herstellungsverfahren:

Die Wirksubstanz und Natriumcyclamat werden in ca. 70 ml Wasser gelöst und Glycerin zugefügt. Man löst die p-Hydroxybenzoesäureester, Anisöl sowie Menthol in Ethanol und fügt diese Lösung unter Rühren der wäßrigen Lösung zu. Abschließend wird mit Wasser auf 100 ml aufgefüllt und schwebeteilchenfrei filtriert.

## Ansprüche

1.) Verwendung von (±)-1-[4-[Ethyl[2-(4-methoxyphenyl)-1-methylethyl]amino]-1-oxobutyl]-N,N-dimethyl-4-piperidincarboxamid und (+)-1-[4-[Ethyl[2-(4-methoxyphenyl)-1-methylethyl]amino]-1-oxobutyl]-N,N-dimethyl-4-piperidincarboxamid bzw. von physiologisch verträglichen Salzen dieser Verbindungen mit anorganischen oder organischen Säuren zur Behandlung von Bradycardien und Bradyarrhythmien.

2.) Verwendung von (±)-1-[4-[Ethyl[2-(4-methoxyphenyl)-1-methylethyl]amino]-1-oxobutyl]-N,N-dimethyl-4-piperidincarboxamid und (+)-1-[4-[Ethyl[2-(4-methoxyphenyl)-1-methylethyl]amino]-1-oxobutyl]-N,N-dimethyl-4-piperidincarboxamid bzw. von physiologisch verträglichen Salzen dieser Verbindungen mit anorganischen oder organischen Säuren zur Herstellung eines Arzneimittels zur Behandlung von Bradycardien und Bradyarrhythmien.